# EUROPÄISCHE PATENTSCHRIFT

(11) **EP 1 810 647 B1**
(45) Veröffentlichungstag und Bekanntmachung des Hinweises auf die Patenterteilung: **11.11.2009**
(21) Anmeldenummer: 06405392.9
(22) Anmeldetag: 12.09.2006
(51) Int. Cl.: A61F 9/01

(54) **Vorrichtung zum Schützen von Gewebe bei der Augenbehandlung**
Apparatus for protecting tissue during eye surgery
Dispositif permettant protection du tissu en chirurgie ophtalmique

(30) Priorität: 23.01.2006 EP 06405030
(43) Veröffentlichungstag der Anmeldung: 25.07.2007
(73) Patentinhaber: Ziemer Holding AG, 2562 Port (CH)
(72) Erfinder: Rathjen, Christian, 28197 Bremen (DE)
(74) Vertreter: Vogel, Dany

(56) Entgegenhaltungen:
- WO-A-03/082162
- US-A1- 2003 223 037
- US-A1- 2004 111 083
- US-A1- 2004 199 149
- US-A1- 2004 243 112

## Beschreibung

### Technisches Gebiet

Die vorliegende Erfindung betrifft eine Vorrichtung zum Schützen von Gewebe bei der Augenbehandlung. Die vorliegende Erfindung betrifft insbesondere eine Vorrichtung zum Schützen von Gewebe bei der Behandlung eines Auges mit einem von einem Lasersystem erzeugten fokussierten gepulsten Laserstrahl.

### Stand der Technik

Bei Femtolasersystemen, die Pulsbreiten von typisch 10fs bis 1000fs (1fs=10⁻¹⁵s) aufweisen, besteht die Besonderheit, dass auch transparente Materialen im Fokus durch nichtlineare Absorption und anschliessende Wechselwirkung (z.B. Photodisruption) bearbeitet werden können. Als Beispiel sei insbesondere das in die Praxis eingeführte operative Schneiden in der Augenhornhaut (Cornea) angeführt. WO 03/032803 beschreibt ein dazu geeignetes System mit einem geschlossenen Rückkopplungskreis zur Positionierung des fokussierten Laserstrahls. Bezeichnend für Femtolasersysteme ist die Möglichkeit der freien Fokusführung im Material, was die Erzeugung beliebiger Schnittmuster ermöglicht. US 5984916 beschreibt ein Beispiel eines Femtolasersystems für die chirurgische Behandlung von Augengewebe. In US 2004/0199149 werden von Schnitten abweichende Wechseiwirkungszonen beschrieben.

Die nichtlineare Absorption ist im allgemeinen nicht vollständig, wodurch bei der Bearbeitung von transparenten Materialen immer ein Teil der eingestrahlten Energie transmittiert wird. Insbesondere beim Auge wird diese Energie von darunter liegenden Gewebeschichten absorbiert. Starke Absorption findet durch den hohen Pigmentanteil in der Retina und der Iris statt.

Die Kombination von freier oder in Grenzen freier Programmierbarkeit der Lasersysteme in Verbindung mit der nur teilweisen Absorption kann bei unsachgemässem Betrieb (z.B. Programmierung) Schädigungen von darunter liegenden Materialbereichen bzw. Gewebeschichten hervorrufen. Am Auge sind z.B. Schäden durch Phototoxizität im kurzwelligem sichtbaren Wellenlängenbereich und Photokoagulation (photothermischer Effekt über den gesamten Wellenlängenbereich) bekannt. Bei der Verwendung von infrarotem Licht kann zum Beispiel eine Kombination beider Effekte auftreten: Phototoxizität durch Weisslichterzeugung im Fokus und Photokoagulation durch nicht absorbiertes Infrarotlicht.

Insbesondere zur Verkürzung von Operationszeiten zeichnet sich vermehrt ein Trend zu Lasersystemen mit höherer Leistung für die Behandlung von Augengewebe ab, was das Gefahrenpotential von ungewollten Gewebeschäden erhöht.

WO 03/082162 beschreibt ein System für ophthalmologische Korrektur, welches eine grafische Benutzeroberfläche zur Auswahl von gesammelten Daten für die Analyse mit historischen Daten aufweist, und welches die Übermittlung von Instruktionen von einem Computer auf das Lasersystem ermöglicht.

US 2003/0223037 beschreibt ein System für die computergesteuerte Behandlung von Comeagewebe mittels der Laserenergie eines Femtolasers. Das System nach US 2003/0223037 ermöglicht insbesondere das Pupillenzentrum sowie die Bewegung des Auges relativ zum Laserstrahl in Echtzeit zu bestimmen und Wellenfrontmessungen des Auges zu speichern.

US 2004/0199149 beschreibt ein Verfahren für die Behandlung von Augengewebe mittels Laserpulsen, in welchem der Chirurg basierend auf biometrischen Linsenmessungen und dem zu verbessernden refraktiven Fehler eine Behandlungsstrategie erstellt. Diese Daten werden von einem Steuerprogramm verwendet, welches die Positionierung des Fokus, die Energiehöhe, Pulsdauer und Frequenz steuert. Die Positionierung des Fokus wird durch ein Scanprogramm so in verschiedene Gebiete der Linse gesteuert, dass unerwünschte Auswirkungen auf das Linsengewebe vermieden werden.

US 2004/0111083 beschreibt ein System für die computerbasierte Planung einer refraktiven Corneabehandlung mittels gepulster Laserenergie, wobei das Behandlungsmuster basierend auf einem Erhitzungsmodel der Cornea bestimmt wird, um lokale Überhitzungen zu vermeiden. Dabei werden insbesondere mehrere Durchgänge geplant und die durchschnittliche Pulsrate basierend auf empirischen Daten und/oder thermodynamischer Analyse bestimmt, wobei die Temperatur unter 44 Grad gehalten wird, um Überhitzung der Cornea zu vermeiden.

Die bekannten Systeme ermöglichen die Erzeugung von Behandlungsmustern, welche den Laserfokus so positionieren und bewegen, dass unerwünschte Auswirkungen auf das Linsengewebe (US 2004/0199149) respektive eine Überhitzung der Cornea (US 2004/0111083) vermieden werden. Bei der Behandlung des Auges mit Femtosekundenlaserpulsen, vor allem im Infrarotbereich, besteht jedoch die Gefahr, dass andere Augenstrukturen, die ausserhalb des zu behandelnden Augengewebes liegen, insbesondere die Iris und die Retina, geschädigt werden.

### Darstellung der Erfindung

Es ist eine Aufgabe der vorliegenden Erfindung eine Vorrichtung zum Schützen von Gewebe bei der Behandlung eines Auges mit einem von einem Lasersystem erzeugten fokussierten gepulsten Laserstrahl vorzuschlagen. Es ist insbesondere eine Aufgabe der vorliegenden Erfindung eine Vorrichtung vorzuschlagen, welche bei der Behandlung eines Auges mit einem von einem Lasersystem erzeugten fokussierten gepulsten Laserstrahl ausserhalb des zu behandelnden Gewebes liegende Augenstrukturen, wie die Iris und die Retina, schützt.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die Vorrichtung zum Schützen von Gewebe bei der Behandlung eines Auges mit einem von einem Lasersystem erzeugten fokussierten gepulsten Laserstrahl, ist eingerichtet, Bearbeitungsdaten zu erfassen, die ein bei der Behandlung durch den Fokus des Laserstrahls bearbeitetes Bearbeitungsgebiet definieren.

Die oben genannten Ziele werden durch die vorliegende Erfindung insbesondere dadurch erreicht, dass die Vorrichtung zum Schützen des Augengewebes überdies mit einem Verarbeitungsmodul versehen ist zum Bereitstellen von Steuerdaten für das Lasersystem basierend auf Eingangsdaten, wobei die Eingangsdaten die Bearbeitungsdaten umfassen, und wobei das Verarbeitungsmodul eingerichtet ist, die Steuerdaten unter Berücksichtigung der Lichtausbreitung im Auge so zu bestimmen, dass die Steuerdaten die Behandlung durch den Fokus des Laserstrahls an Positionen innerhalb des Bearbeitungsgebiets zeitlich jeweils so begrenzen, dass sich aus der Behandlung durch den Laserstrahl ergebende Gewebeschäden, in ausserhalb des Bearbeitungsgebiets liegenden Augenstrukturen, im wesentlichen vermieden werden. Das Lasersystem ist beispielsweise ein Femtolasersystem. Die Steuerdaten werden insbesondere so bestimmt, dass im Gewebe eine mittlere Bestrahlungsstärke reduziert wird und maximale Temperaturen im Gewebe von ausserhalb des Bearbeitungsgebiets liegenden Augenstrukturen, insbesondere in der Iris und der Retina, reduziert werden. Die Steuerdaten bestimmen insbesondere Bewegungen des Fokus des Laserstrahls innerhalb des Bearbeitungsgebiets so, dass die mittlere Bestrahlungsstärke reduziert wird und die maximalen Temperaturen im Gewebe von ausserhalb des Bearbeitungsgebiets liegenden Augenstrukturen, insbesondere in der Iris und der Retina, reduziert werden. Zusätzlich oder alternativ aktivieren und deaktivieren die Steuerdaten den Laserstrahl zeitweise so, dass die mittlere Bestrahlungsstärke reduziert wird und die maximalen Temperaturen im Gewebe von ausserhalb des Bearbeitungsgebiets liegenden Augenstrukturen, insbesondere in der Iris und der Retina, reduziert werden. Beim Erzeugen der Steuerdaten werden insbesondere potentielle Gewebeschäden durch Überhitzung, Phototoxizität und/oder Photokoagulation berücksichtigt. Durch die Bereitstellung von Steuerdaten, die die Behandlung durch den Fokus des Laserstrahls an Positionen innerhalb des Bearbeitungsgebiets zeitlich begrenzen, können Schädigungen von Gewebeschichten ausserhalb des Bearbeitungsgebiets, die die eingestrahlte Energie absorbieren, vermieden werden. Durch die Berücksichtigung der Lichtausbreitung im Auge kann das Licht respektive die Energie bestimmt werden, die in andere ausserhalb des Bearbeitungsgebiets liegende Augenstrukturen eingestrahlt wird. Somit können Steuerdaten bereitgestellt werden, welche die Einstrahlung von Licht und somit die Lichtabsorption in diese nicht zu behandelnden Augenstrukturen (d.h. Augenteile, welche keinen Teil des Bearbeitungsgebiets umfassen) zeitlich begrenzen und Gewebeschäden in diesen Augenstrukturen vermeiden. Zum Beispiel können bei der Behandlung der Cornea oder der Linse Schädigungen an ausserhalb der Cornea respektive Linse liegenden Augenstrukturen vermieden werden, insbesondere in der Iris und der Retina, welche auf Grund ihrer hohen Pigmentierung eine hohe Absorption aufweisen.

In einer bevorzugten Ausführungsvariante werden durch die Vorrichtung Laserparameter des Lasersystems gespeichert und die Steuerdaten werden basierend auf den Eingangsdaten erzeugt, wobei die Eingangsdaten zudem die Laserparameter umfassen. Die Laserparameter umfassen Pulsenergie, Pulsbreite, maximale Pulsintensität (je nach Pulsform ergeben sich unterschiedliche Werte der Pulsintensität, bei sonst gleicher Pulsbreite und Pulsenergie), Pulsrate, Wellenlänge, Fokusgrösse, mittlere Laserleistung, numerische Apertur und/oder Scanmuster des Lasersystems. Die Berücksichtigung der Laserparameter ermöglicht die Steuerdaten spezifisch für das eingesetzte Lasersystem bereitzustellen und damit den Einsatz der Laserenergie zeitlich und energetisch optimal zu gestalten.

In einer bevorzugten Ausführungsvariante werden durch die Vorrichtung Augenabmessungen gespeichert und die Steuerdaten werden basierend auf den Eingangsdaten erzeugt, wobei die Eingangsdaten zudem die Augenabmessungen umfassen. Die Augenabmessungen definieren ein Augenmodell, insbesondere eine optische Geometrie des Auges, und umfassen Angaben über Grösse und relative Position von Augenstrukturen, wobei die Augenstrukturen den Bulbus oculi, die Cornea, Linse, Retina, Pupille und/oder Iris umfassen. Die Berücksichtigung der Augenabmessungen ermöglicht die Steuerdaten spezifisch für das zu behandelnde Auge bereitzustellen und damit den Einsatz der Laserenergie zeitlich und energetisch optimal zu gestalten.

In einer Ausführungsvariante werden durch die Vorrichtung empirische Daten über Belastungsgrenzen von Augenstrukturen gespeichert, und die Steuerdaten werden basierend auf den Eingangsdaten erzeugt, wobei die Eingangsdaten zudem die Belastungsgrenzen umfassen. Die empirischen Daten umfassen beispielsweise Belastungsgrenzen von Augenstrukturen für verschiedene Laserparameter. Durch die Speicherung von empirischen Belastungsgrenzen ist es möglich die Vorrichtung einfach mit neuen Erfahrungswerten zu konfigurieren.

In einer Ausführungsvariante umfasst die Vorrichtung ein Modellierungsmodul, welches eingerichtet ist, basierend auf den Eingangsdaten, sich aus der Behandlung durch den Laserstrahls ergebende Belastungen der Augenstrukturen zu bestimmen, und die Steuerdaten werden basierend auf diesen Belastungen und gespeicherten Belastungsgrenzen bestimmt. Durch die Modellierung und Berechnung der erwarteten Belastungen der Augenstrukturen können die Steuerdaten möglichst genau an die erwarteten Schadensrisiken angepasst werden. Das Augenmodell umfasst in einer Variante auch den Augenstrukturen zugeordnete Absorptionskoeffizienten, die bei der Bestimmung der sich aus der Behandlung durch den Laserstrahls ergebende Belastungen der Augenstrukturen berücksichtigt werden.

In einer Ausführungsvariante werden auf den Steuerdaten basierende Steuersignale an das Lasersystem übertragen. In einer alternativen Ausführungsvariante werden die Steuerdaten zur Speicherung an das Lasersystem zu übertragen. Die Speicherung der Steuerdaten ermöglicht eine automatische Steuerung des Lasersystems basierend auf den vorgängig durch die Sicherheitsvorrichtung bereitgestellten Steuerdaten. Die Übertragung der Steuersignale ermöglicht eine Echtzeitsteuerung des Lasersystems durch die Sicherheitsvorrichtung.

In einer bevorzugten Ausführungsvariante werden die Bearbeitungsdaten direkt vom Lasersystem entgegengenommen. Die Erfassung der Bearbeitungsdaten direkt vom Lasersystem ermöglicht eine Echtzeitkontrolle und -überwachung des Bearbeitungsvorgangs am Auge und damit eine Erhöhung der Sicherheit.

In einer Ausführungsvariante werden auf den Steuerdaten basierende Bearbeitungsmuster und/oder Bearbeitungsinstruktionen auf einer Anzeige angezeigt, welche Bearbeitungsmuster und/oder Bearbeitungsinstruktionen einen Bewegungsablauf des Fokus des Laserstrahls bei der Behandlung des Bearbeitungsgebiets definieren.

Neben der Vorrichtung zum Schützen von Gewebe bei der Behandlung eines Auges mit einem von einem Lasersystem erzeugten fokussierten gepulsten Laserstrahl bezieht sich die vorliegende Erfindung zudem auf ein Computerprogrammprodukt mit Computerprogrammcodemitteln zur Steuerung eines oder mehrerer Prozessoren, insbesondere ein Computerprogrammprodukt mit einem computerlesbaren Medium das die Computerprogrammcodemittel umfasst. Die Computerprogrammcodemittel Steuern die Prozessoren derart, dass sie Steuerdaten bereitstellen, die geeignet sind ein Lasersystem so zu steuern, dass bei der Behandlung eines Auges mit einem vom Lasersystem erzeugten fokussierten gepulsten Laserstrahl Gewebeschäden, an ausserhalb des Bearbeitungsgebiets liegenden Augenstrukturen, im wesentlichen vermieden werden.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch ein Lasersystem bei der Behandlung eines Auges mittels eines fokussierten gepulsten Laserstrahls, sowie eine Vorrichtung zum Schützen von Gewebe des Auges bei der Behandlung darstellt.
Figuren 2a, 2b und 2c zeigen jeweils ein Blockdiagramm, welches schematisch eine mögliche Zusammensetzung eines Datenerfassungsmoduls der Vorrichtung darstellt.
Figuren 3a, 3b und 3c zeigen jeweils ein Blockdiagramm, welches schematisch eine mögliche Konfiguration eines Verarbeitungsmoduls der Vorrichtung darstellt.
Figur 4 zeigt ein Flussdiagramm, das schematisch mögliche Verfahrensabläufe zum Schützen des Gewebe bei der Behandlung des Auges durch den fokussierten gepulsten Laserstrahl des Lasersystems darstellt.

### Wege zur Ausführung der Erfindung

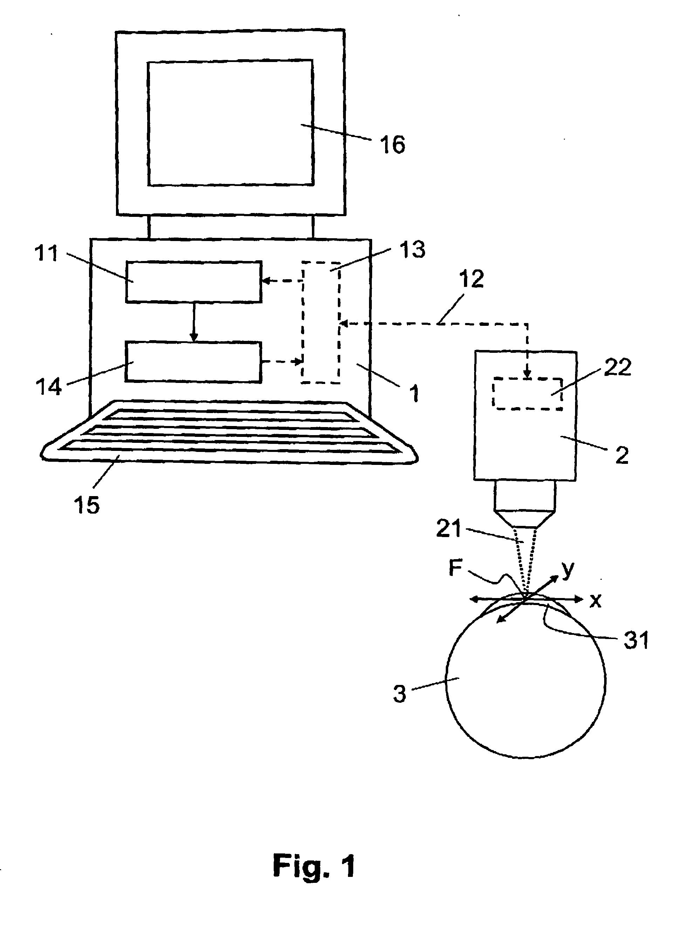

In der Figur 1 bezeichnet das Bezugszeichen 2 ein Lasersystem, das eingerichtet ist, einen fokussierten gepulsten Laserstrahl 21 zu erzeugen und mittels des Laserstrahls 21 ein Auge 3 zu bearbeiten. Wie in der Figur 1 schematisch dargestellt ist, ist das Lasersystem 2 eingerichtet, den Fokus F (Brennpunkt) des Laserstrahls 21 mindestens in zwei Dimensionen x, y eines (zusammenhängenden oder nicht zusammenhängenden) Bearbeitungsgebiets im oder auf dem Gewebe 31 des Auges 3 zu bewegen. In einer Ausführungsvariante ist das Lasersystem 2 zudem eingerichtet, den Fokus F auch in einer dritten, zu den zwei Dimensionen x, y normalen Richtung zu bewegen. Das Lasersystem 2 umfasst vorzugsweise einen Femtolaser zur Erzeugung von Femtosekundenlaserpulsen, die Pulsbreiten von typisch 100fs bis 1000fs (1fs=10⁻¹⁵s) aufweisen. In einer Ausführungsvariante umfasst das Lasersystem 2 ein Kommunikationsmodul 22 für den eindirektionalen oder bidirektionalen Datenaustausch über die Kommunikationsschnittstelle 12 mit einem Kommunikationsmodul 13 der Vorrichtung 1. Die Kommunikationsschnittstelle 12 ist beispielsweise eine drahtgebundene Schnittstelle, z.B. eine RS-232, RS-422 oder RS-485 Schnittstelle, oder eine kontaktlose Schnittstelle, z.B. eine Infrarotschnittstelle (z.B. IrDA) oder eine Funkschnittstelle (z.B. Bluetooth).

In der Figur 1 bezeichnet das Bezugszeichen 1 eine Vorrichtung zum Schützen des Gewebes 31 bei der Behandlung des Auges 3 mittels des vom Lasersystem 2 erzeugten Laserstrahls 21. Die Vorrichtung 1 umfasst vorzugsweise einen funktionsfähigen, programmierbaren Prozessor sowie damit verbundene Daten- und Programmspeicher, oder ein anderes elektronisches Logikmodul. Vorzugsweise ist die Vorrichtung 1 mit Bedienungselementen 15, z.B. eine Tastatur, eine Positionierungsvorrichtung (Computermaus, Joystick, Trackball, Touch Pad, oder ähnliche) und/oder eine Sprachsteuereinheit, sowie mit einer Anzeige 16 ausgestattet oder verbunden. Die Vorrichtung 1 ist beispielsweise als Personal Computer oder als speziell konfiguriertes Elektronikmodul ausgeführt. In einer Ausführungsvariante sind die Vorrichtung 1 und das Lasersystem 2 in ein gemeinsames Gehäuse integriert. Neben dem bereits erwähnten Kommunikationsmodul 13 umfasst die Vorrichtung 1 ein Datenerfassungsmodul 11 und ein Verarbeitungsmodul 14. Das Verarbeitungsmodul 14 ist für den Datenzugriff mit dem Datenerfassungsmodul 11 verbindbar. Wie später detaillierter beschrieben wird, sind das Datenerfassungsmodul 11, für die Entgegennahme von Daten vom Lasersystem 2, und das Verarbeitungsmodul 14, für die Übermittlung von Daten an das Lasersystem 2, mit dem Kommunikationsmodul 13 verbindbar. Die Bedienung, Steuerung und Konfiguration des Verarbeitungsmoduls 14 und des Datenerfassungsmoduls 11 durch den Benutzer erfolgt über die Bedienungselemente 15 und die Anzeige 16, beispielsweise über eine graphische Benuizerschnittstelle. Die funktionalen Teile des Verarbeitungsmoduls 14 und des Datenerfassungsmoduls 11 sind vorzugsweise als programmierte Softwaremodule oder Softwareteile ausgeführt. Der Computerprogrammcode ist Teil eines Computerprogrammprodukts und ist vorzugsweise in der Vorrichtung 1 gespeichert, auf einem computerlesbaren Datenträger, der fest oder entfernbar mit der Vorrichtung 1 verbunden ist. Der Fachmann wird verstehen, dass die funktionalen Teile des Verarbeitungsmoduls 14 und des Datenerfassungsmoduls 11 auch teilweise oder vollständig hardwaremässig ausgeführt werden können.

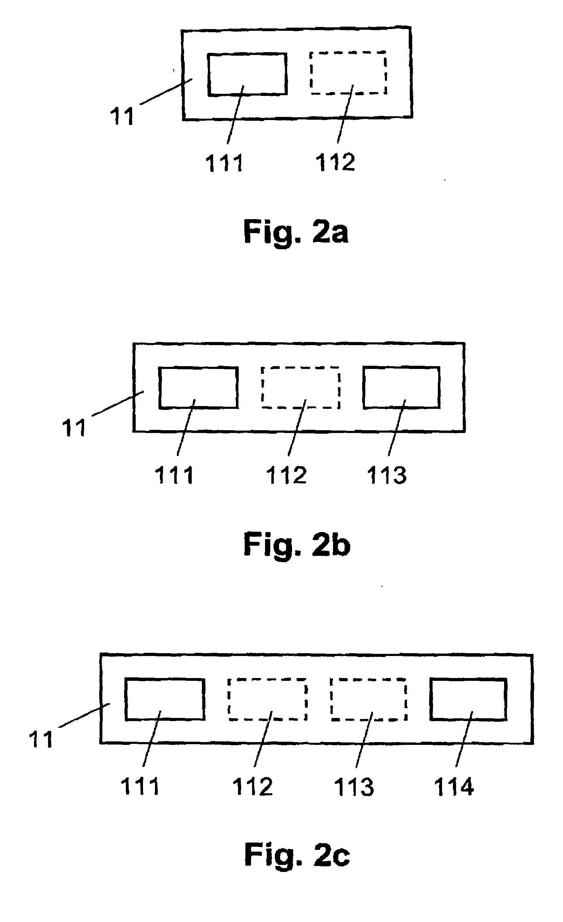

Wie in den Figuren 2a, 2b und 2c schematisch dargestellt ist, ist das Datenerfassungsmodul 11 je nach Ausführungsvariante unterschiedlich konfiguriert. In den dargestellten Ausführungsvarianten umfasst das Datenerfassungsmodul 11 jeweils ein Bearbeitungsdatenmodul 111 zum Erfassen und Speichern von Bearbeitungsdaten, die ein (zusammenhängendes oder nicht zusammenhängendes) Bearbeitungsgebiet definieren, das bei der Behandlung durch den Fokus F des Laserstrahls 21 bearbeitet wird. Die Bearbeitungsdaten umfassen vorgegebene Bearbeitungsdaten, die vom Benutzer angegeben werden, und/oder aktuelle Bearbeitungsdaten, die vom Bearbeitungsdatenmodul 111 über die Kommunikationsschnittstelle 12 vom Lasersystem 2 erfasst werden. Die vorgegebenen Bearbeitungsdaten werden vom Benutzer beispielsweise in Form von Koordinaten, Form- und Grössenangaben, und/oder Positionsangaben definiert. Die aktuellen Bearbeitungsangaben umfassen beispielsweise Positionswerte (Koordinaten) des Fokus F des Laserstrahls 21, die durch das Bearbeitungsdatenmodul 111 jeweils zugeordnet zu Zeitwerten, die den aktuellen Zeitpunkt des Datenempfangs oder der Positionserfassung im Lasersystem 2 angeben, gespeichert werden. Wenn der Fokus mit einer bekannten und konstanten Geschwindigkeit bewegt wird, kann auf die Erfassung der Zeitwerte verzichtet werden, solange die Sequenz der erfassten Positionswerte gewahrt bleibt.

Wie in den Figuren 2a, 2b und 2c angedeutet wird, umfasst das Datenerfassungsmodul 11 in einer Ausführungsvariante zusätzlich zum Bearbeitungsdatenmodul 111 ein Laserdatenmodul 112 zum Erfassen und Speichern von Laserparametern des Lasersystems 2. Die Laserparameter umfassen technische Kenndaten des Lasersystems 2. Die Laserparameter umfassen beispielsweise Datenwerte, die die Pulsenergie, die Pulsbreite, die Pulsrate, die Wellenlänge, die Fokusgrösse, die mittlere Laserleistung, die numerische Apertur und/oder das Scanmuster angeben. In einer Ausführungsvariante umfassen die Laserparameter zudem Angaben über Kontaktkörper, beispielsweise Angaben über Dicke, Form und/oder Transmissionscharakteristik. Die Laserparameter werden entweder vom Benutzer angegeben, über die Kommunikationsschnittstelle 12 vom Lasersystem 2 gelesen oder von einer anderen externen Datenquelle bezogen. Die Laserparameter können auch fest programmiert sein.

Wie in der Figur 2b schematisch dargestellt ist, umfasst das Datenerfassungsmodul 11 in einer Ausführungsvariante zusätzlich zum Bearbeitungsdatenmodul 111 und dem optionalen Laserdatenmodul 112 ein Augendatenmodul 113 zum Erfassen und Speichern von Augenabmessungen. Die Augenabmessungen umfassen Angaben über Grösse und relative Position von Augenstrukturen im undeformierten und/oder deformierten Zustand (z.B. verschiedene deformierte Zustände der Augenstrukturen abhängig von verschiedenen Kontaktkörpern). Die Augenstrukturen umfassen Cornea, Linse, Retina, Pupille, Bulbus oculi und/oder Iris. Die Augenabmessungen werden entweder vom Benutzer angegeben, über die Kommunikationsschnittstelle 12 vom mit entsprechenden Messvorrichtungen ausgestatteten Lasersystem 2 gelesen oder von einer anderen externen Messvorrichtung bezogen. Augenabmessungen für ein generisches Auge können auch fest programmiert sein. In einer Ausführungsvariante erfasst und speichert das Augendatenmodul 113 zudem jeweils den Augenstrukturen zugeordnete spezifische Lichtabsorptionskoeffizienten.

Wie in der Figur 2c schematisch dargestellt ist, umfasst das Datenerfassungsmodul 11 in einer Ausführungsvariante zusätzlich zum Bearbeitungsdatenmodul 111, dem optionalen Laserdatenmodul 112 und dem optionalen Augendatenmodul 113 ein Referenzdatenmodul 114 zum Erfassen und Speichern von empirischen Daten über Belastungsgrenzen der Augenstrukturen (die z.B. direkt mit dem Lasersystem gewonnen wurden) und von empirischen Daten über Gewebeschäden. Vorzugsweise umfassen die empirischen Daten Belastungsgrenzen von Augenstrukturen für verschiedene Laserparameter. Die Belastungsgrenzen von Augenstrukturen sind beispielsweise für verschiedene definierte Kombinationen von Werten der Laserparameter oder definierte Konfigurationen des Lasersystems 2 erfasst Die Belastungsgrenzen werden beispielsweise in Form einer maximalen mittleren Bestrahlungsstärke und/oder maximalen Bestrahlungszeit bei bekannter Bestrahlung angegeben. Die mittlere Bestrahlungsstärke wird beispielsweise hinsichtlich einer Gesamtbearbeitungszeit, über das gesamte Bearbeitungsgebiet, und/oder einer Lokalbearbeitungszeit, über ein definiertes Teilgebiet, spezifiziert. Die empirischen Daten werden entweder vom Benutzer angegeben, über die Kommunikationsschnittstelle 12 vom Lasersystem 2 gelesen oder von einer anderen externen Datenquelle bezogen. Die Belastungsgrenzen können auch fest programmiert sein.

Das Verarbeitungsmodul 14 ist eingerichtet, basierend auf Eingangsdaten, die mindestens die Bearbeitungsdaten zur Bestimmung des Bearbeitungsgebiets umfassen, Steuerdaten für das Lasersystem 2 bereitzustellen. Die Steuerdaten umfassen Instruktionen für das Lasersystem 2 und/oder für den Benutzer des Lasersystems 2. Die Instruktionen betreffen beispielsweise das Aktivieren/Deaktivieren des Laserstrahls 21, bestimmte Einstellungen des Lasersystems wie die Bestrahlungsstärke oder Pulsfrequenz, und/oder das Bewegen des Fokus in eine bestimmte Richtung respektive an eine bestimmte Position- Das Verarbeitungsmodul 14 bestimmt die Steuerdaten so, dass die Behandlung durch den Fokus F im Bearbeitungsgebiet zeitlich so begrenzt ist, dass Gewebeschäden in Augenstrukturen, die ausserhalb des Bearbeitungsgebiets liegen, im wesentlichen vermieden werden. Bei der Bearbeitung der Comea oder der Linse werden beispielsweise Gewebeschäden in der Iris oder der Retina vermieden. Das Verarbeitungsmodul 14 ist in einer Variante zudem so eingerichtet, dass es die Steuerdaten so bestimmt, dass die fokussierte Projektion der Laserpulse zeitlich so begrenzt wird, dass Gewebeschäden ausserhalb des Bearbeitungsgebiets und insbesondere auch in unmittelbarer Nähe des Bearbeitungsgebiets im wesentlichen vermieden werden. Beispielsweise sollen bei der Bearbeitung der Cornea Schäden am unmittelbar benachbarten stromaien Gewebe und/oder im darüber liegenden Epithelgewebe vermieden werden. Die Steuerdaten werden so bestimmt, dass die mittlere Bestrahlungsstärke im Gewebe reduziert wird und maximale Temperaturen im Gewebe ausserhalb des Bearbeitungsgebiets reduziert und beispielsweise auf definierte Höchstwerte begrenzt werden. Die Steuerdaten werden beispielsweise so bestimmt, dass sie für die Bewegungen des Fokus innerhalb des Bearbeitungsgebiets ein Bewegungsmuster bestimmen, bei welchem die mittlere Bestrahlungsstärke im Gewebe reduziert wird und maximale Temperaturen im Gewebe ausserhalb des Bearbeitungsgebiets reduziert werden. Zudem oder als Alternative werden die Steuerdaten so bestimmt, dass sie den Laserstrahl zeitweise so aktivieren und deaktivieren, dass die mittlere Bestrahlungsstärke reduziert wird und die maximalen Temperaturen im Gewebe ausserhalb des Bearbeitungsgebiets reduziert werden. Die mittlere Bestrahlungsstärke wird für das Zeitfenster einer Behandlung in einem lokalen, zusammenhängenden Teilbearbeitungsgebiet oder für das Zeitfenster einer Gesamtbehandlung über das gesamte Bearbeitungsgebiet bestimmt. Beim Erzeugen der Steuerdaten berücksichtigt das Verarbeitungsmodul 14 potentielle Gewebeschäden durch Überhitzung, Phototoxizität und/oder Photokoagulation. Das Verarbeitungsmodul 14 bezieht die Eingangsdaten vom Datenerfassungsmodul 11 und/oder vom Benutzer.

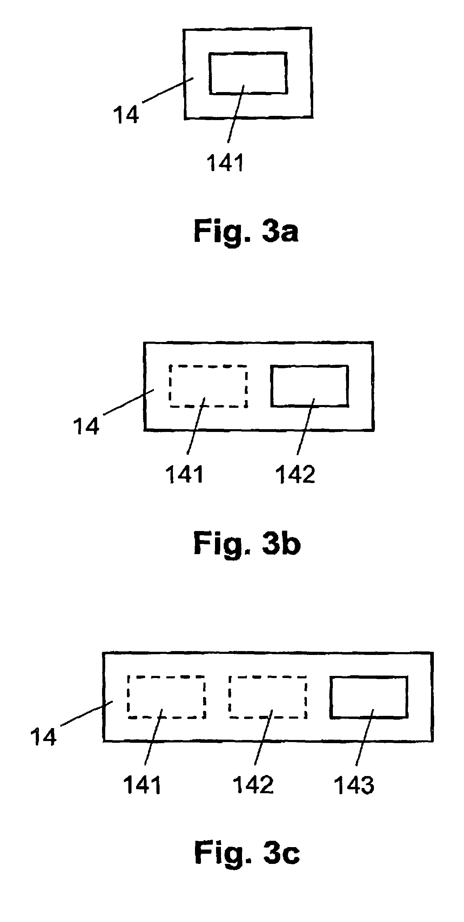

Wie in den Figuren 3a, 3b und 3c schematisch dargestellt ist, ist das Verarbeitungsmodul 14 je nach Ausführungsvariante unterschiedlich konfiguriert. In der einfachsten Ausführungsvariante ist das Verarbeitungsmodul 14 eingerichtet, die Steuerdaten einzig in Abhängigkeit des Bearbeitungsgebiets auf Grund der Bearbeitungsdaten bereitzustellen, unabhängig von Laserparametern, beispielsweise für einen definierten Standardtyp des Lasersystems 2, und unabhängig von Augenabmessungen, für ein generisches Auge mit durchschnittlichen Augenabmessungen und Lichtabsorptionskoeffizienten. Im einfachsten Fall sind vordefinierte Steuerdaten unterschiedlichen Bearbeitungs- oder Bewegungsmustern zugeordnet gespeichert. In flexibleren Ausführungsvarianten berücksichtigt das Verarbeitungsmodul 14 für die Bereitstellung der Steuerdaten zudem auch die Laserparameter des Lasersystems 2, die Augenabmessungen des Auges 3, die Lichtabsorptionskoeifizienten der verschiedenen Augenstrukturen und/oder die empirischen Daten über die Belastungsgrenzen der Augenstrukturen. Zum Beispiel sind vordefinierte Steuerdaten unterschiedlichen Bearbeitungs- oder Bewegungsmustern sowie Laserparametern und/oder Augenabmessungen zugeordnet gespeichert, das heisst für unterschiedliche Kombinationen von Bearbeitungs- oder Bewegungsmustern, Laserparametern und/oder Augenabmessungen ergeben sich andere vordefinierte Steuerdaten. In der flexibelsten Ausführungsvariante umfasst das Verarbeitungsmodul 14, wie in den Figuren 3a, 3b und 3c angedeutet, ein Modellierungsmodul 141, das eingerichtet ist, basierend auf den Eingangsdaten, die mindestens die Bearbeitungsdaten umfassen, Belastungen der Augenstrukturen zu bestimmen, die sich aus der Behandlung durch den Laserstrahl 21 ergeben. Die Belastungen der Augenstrukturen werden auf Grund der Energie bestimmt, die bei der Behandlung des Bearbeitungsgebiets durch einen Standardlaser während einer bestimmten Einwirkungszeit erwartungsgemäss in den Augenstrukturen eines generischen Auges mit durchschnittlichen Augenabmessungen absorbiert wird. Wenn das Datenerfassungsmodul 11 ein Laserdatenmodul 112 umfasst, umfassen die Eingangsdaten auch die Laserparameter des Lasersystems 2 und das Modellierungsmodul 141 bestimmt die Belastungen der Augenstrukturen, die sich aus der Behandlung des durch die Bearbeitungsdaten bestimmten Bearbeitungsgebiets mittels des durch die Laserparameter bestimmten Lasersystems 2 ergeben. Wenn das Datenerfassungsmodul 11 ein Augendatenmodul 113 umfasst, umfassen die Eingangsdaten auch die Augenabmessungen des Auges 3 und das Modellierungsmodul 141 bestimmt die Belastungen der Augenstrukturen unter Berücksichtigung dieser Augenabmessungen. In der flexibelsten Ausführungsvariante werden die Belastungen der Augenstrukturen auf Grund der Energie bestimmt, die bei der Behandlung des Bearbeitungsgebiets durch das durch die Laserparameter definierte Lasersystem 2 während einer bestimmten Einwirkungszeit erwartungsgemäss in den durch die Augenabmessungen definierten Augenstrukturen absorbiert wird. In einer Ausführungsvariante bestimmt das Modellierungsmodul 141 die Belastungen der Augenstrukturen unter Berücksichtigung der empirischen Daten über Gewebeschäden. Das Modellierungsmodul 141 bestimmt die Belastungen der Augenstrukturen unter Anwendung von physikalischen Gesetzen zur Lichtausbreitung, Wärmeleitung und/oder zum Absorptionsverhalten, beispielsweise unter Berücksichtigung der individuellen Lichtabsorptionskoeffizienten der verschiedenen Augenstrukturen. Das Modeflierungsmodul 141 ist insbesondere eingerichtet die Ausbreitung des bei der Behandlung durch den Laserstrahl 21 eingestrahlten Lichts im Auge 3 (rechnerisch und/oder tabellarisch) zu ermitteln. Es wird der definierte Standardtyp des Lasersystems 2 oder ein durch die Laserparameter definiertes Lasersystem 2 angenommen. Die Lichtausbreitung wird auf der Grundlage eines generischen Augenmodells, mit durchschnittlichen Abmessungen, Distanzen und Eigenschaften (z.B. Streuung, Brechung, Reflexion) der Augenstrukturen, oder eines durch die erfassten Augenabmessungen bestimmten Augenmodells bestimmt. Dabei werden für die Projektion der fokussierten Laserpulse auf einen Fokus F innerhalb des Bearbeitungsgebiets jeweils die Lichtausbreitung im Auge und insbesondere das in die ausserhalb des Bearbeitungsgebiets liegenden Augenstrukturen eingestrahlte Licht bestimmt. Das Augenmodell definiert beispielsweise den Anteil (zB. eine Prozentangabe) der an einer bestimmten Fokusposition eingestrahlten Lichtrespektive Energiemenge, die in eine bestimmte Augenstruktur und/oder an eine bestimmte Stelle dieser Augenstruktur eingestrahlt wird. Durch die Integration der durch mehrere Laserpulse in die ausserhalb des Bearbeitungsgebiets liegenden Augenstrukturen eingestrahlten Lichtrespektive Energiemenge wird die Belastung, insbesondere die Wärmebelastung, der Augenstrukturen bestimmt. Vorzugsweise wird dabei die Wärmeleitung und/oder das Absorptionsverhalten des Gewebes der Augenstrukturen berücksichtigt. Zum Beispiel wird bei der Behandlung der Cornea die Licht- respektive Energiemenge bestimmt, die auf Grund der in die Cornea projizierten Laserpulse in/auf die Iris und/oder Retina eingestrahlt wird. Darauf basierend wird die Wärmebelastung der Iris respektive der Retina bestimmt. Es werden beispielsweise regionale Durchschnittswerte, punktuelle Maximalwerte, punktuelle Individualwerte und/oder regionale Werteverteilungen der Lichtmenge, Energiemenge und/oder Wärmebelastung in den Augenstrukturen ermittelt.

Das Verarbeitungsmodul 14 bestimmt die Steuerdaten für das Lasersystem 2 basierend auf den Belastungen der Augenstrukturen, die durch das Modellierungsmodul 141 bestimmt werden. Dazu berücksichtigt das Verarbeitungsmodul 14 vorzugsweise die im Referenzdatenmodul 114 gespeicherten Daten über die Belastungsgrenzen der Augenstrukturen und/oder die empirischen Daten über Gewebeschäden.

Wie in der Figur 3b schematisch dargestellt ist, umfasst das Verarbeitungsmodul 14 in einer Ausführungsvariante zusätzlich zum optionalen Modellierungsmodul 141 ein Signalisierungsmodul 142. Das Signalisierungsmodul 142 ist eingerichtet, basierend auf den Steuerdaten Steuersignale zu erzeugen und über das Kommunikationsmodul 13 an das Lasersystem 2 zu übertragen.

Wie in der Figur 3c schematisch dargestellt ist, umfasst das Verarbeitungsmodul 14 in einer Ausführungsvariante zusätzlich zum optionalen Modellierungsmodul 141 und zum optionalen Signalisierungsmodul 142 ein Planungsmodul 143. Das Planungsmodul 143 ist eingerichtet, basierend auf den Steuerdaten Bearbeitungsmuster auf der Anzeige 16 anzuzeigen, welche Bearbeitungsmuster den geplanten und/oder tatsächlichen (aktuellen) Bewegungsablauf des Fokus F bei der Behandlung des Bearbeitungsgebiets darstellen. In einer Ausführungsvariante ist das Planungsmodul 143 eingerichtet die Steuerdaten für geplante Bewegungsabläufe (d.h. für geplante Behandlungen) über das Kommunikationsmodul 13 zur Speicherung an das Lasersystem 2 zu übertragen. Bei der Darstellung aktueller Bewegungsabläufe werden beispielsweise auch entsprechende Steuersignale mittels des Signalisierungsmoduls 142 an das Lasersystem 2 übertragen.

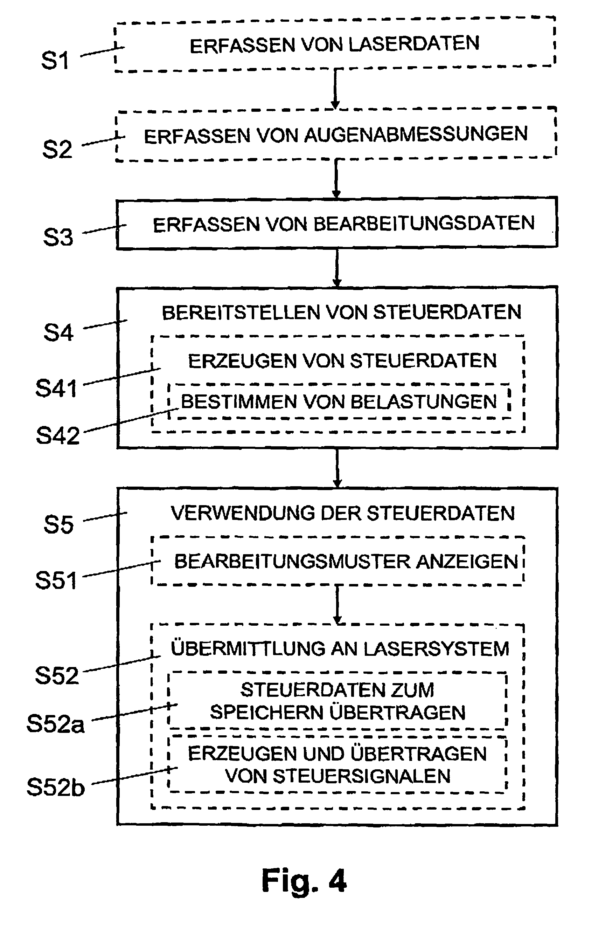

In den folgenden Abschnitten werden mit Bezug auf die Figur 4 mögliche Verfahrensschritte beschrieben zum Schützen von Gewebe 31 mittels der Vorrichtung 1 bei der Behandlung des Auges 3 durch den Laserstrahl 21.

Im optionalen Schritt S1 erfasst und speichert das Laserdatenmodul 112 die Laserparameter des Lasersystems 2.

Im optionalen Schritt S2 erfasst und speichert das Augendatenmodul 113 die Augenabmessungen des Auges 3.

Im Schritt S3 erfasst und speichert das Bearbeitungsdatenmodul 111 die aktuellen und/oder vorgegebenen, geplanten Bearbeitungsdaten des aktuellen respektive geplanten Bearbeitungsgebiets.

Im Schritt S4 stellt das Verarbeitungsmodul 14 die Steuerdaten basierend auf den Bearbeitungsdaten, den Laserparametern des Lasersystems 2, den Augenabmessungen des Auges 3 und/oder den empirischen Daten über die Belastungsgrenzen der Augenstrukturen bereit, In der flexibelsten Ausführungsvariante bestimmt das Modellierungsmodul 141 im Schritt S42 die Belastungen der Augenstrukturen, die sich durch eine Bearbeitung entsprechend den Bearbeitungsdaten ergeben, und das Verarbeitungsmodul 14 erzeugt die Steuerdaten im Schritt S41 basierend auf diesen Belastungen und den gespeicherten Belastungsgrenzen der Augenstrukturen.

Im Schritt S5 werden die Steuerdaten von der Vorrichtung 1 verwendet. Falls die Vorrichtung 1 als Planungsvarrichtung oder Überwachungsvorrichtung eingerichtet ist, werden die Steuerdaten im Schritt S51 als Bearbeitungsmuster und/oder Bearbeitungsinstruktionen auf der Anzeige 16 angezeigt. Die angezeigten Bearbeitungsmuster und/oder Bearbeitungsinstruktionen bestimmen den Bewegungsablauf des Fokus F bei der Behandlung des Bearbeitungsgebiets. In der einfachsten Form sind die Steuerdaten auf der Anzeige darstellbare Instruktionen an den Benutzer für die Steuerung und Bedienung des Lasersystems 2, beispielsweise Instruktionen wie Stopp, Deaktivieren, respektive Weiterfahren, Aktivieren, Bearbeitungsmuster Setzen und/oder Ausführen. Die Überwachungsvorrichtung eignet sich insbesondere für die Unterstützung eines Benutzers bei der Ausführung einer halbautomatischen Behandlung, in der der Benutzer das Lasersystem manuell mittels eines Bedienungselements, z.B. ein Joystick, steuert. In einer sichereren Ausführungsvariante werden die Steuerdaten im Schritt S52 zur Steuerung des Lasersystems 2 an das Lasersystem 2 übertragen, beispielsweise zur Steuerung des Bewegungsablaufs des Fokus F und/oder der Aktivierung des Laserstrahls 21. Das durch die Steuerdaten definierte Bewegungsmuster des Fokus F wird beispielsweise auf der Anzeige 16 dargestellt und die Steuerdaten werden im Schritt S52a zur Speicherung an das Lasersystem 2 übertragen, wo sie das Lasersystem 2 steuern. Falls die Vorrichtung 1 als Echtzeitsteuervorrichtung eingerichtet ist, werden im Schritt S52b basierend auf den Steuerdaten Steuersignale erzeugt und zur Echtzeitsteuerung des Lasersystems 2 an das Lasersystem 2 übermittelt. In dieser Ausführungsvariante, werden vorzugsweise zudem aktuelle Bearbeitungsdaten, die das aktuell bearbeitete Bearbeitungsgebiet definieren, vom Lasersystem 2 an die Vorrichtung 1 übertragen (Rückkopplung), z.B. eine Sequenz aktueller Positionen. Eine derartige Rückmeldung der aktuellen Bearbeitungsdaten vom Lasersystem 2 an die Vorrichtung 1 ist beispielsweise auch für die oben beschriebene Überwachungsvorrichtung für den halbautomatischen Einsatz von Vorteil.

## Patentansprüche

1. Vorrichtung (1) zum Schützen von Gewebe bei der Behandlung eines Auges (3) mit einem von einem Lasersystem (2) erzeugten fokussierten gepulsten Laserstrahl (21), umfassend ein Bearbeitungsdatenmodul (111) zum Erfassen von Bearbeitungsdaten, die ein bei der Behandlung durch den Fokus (F) des Laserstrahls (21) bearbeitetes Bearbeitungsgebiet definieren,
wobei die Vorrichtung ein Verarbeitungsmodul (14) umfasst zum Bereitstellen von Steuerdaten für das Lasersystem (2) basierend auf Eingangsdaten, wobei die Eingangsdaten die Bearbeitungsdaten umfassen, **dadurch gekennzeichnet, dass** das Verarbeitungsmodul (14) eingerichtet ist, für die Projektion eines Laserpulses auf den Fokus (F) innerhalb des Bearbeitungsgebiets die Lichtausbreitung im Auge (3) und das in ausserhalb des Bearbeitungsgebiets liegenden Augenstrukturen eingestrahlte Licht zu bestimmen, und die Steuerdaten unter Berücksichtigung der Lichtausbreitung im Auge so zu bestimmen, dass die Steuerdaten die Behandlung durch den Fokus (F) des Laserstrahls (21) an Positionen innerhalb des Bearbeitungsgebiets zeitlich jeweils so begrenzen, dass sich aus der Behandlung durch den Laserstrahl (21) ergebende Gewebeschäden, in ausserhalb des Bearbeitungsgebiets liegenden Augenstrukturen, im wesentlichen vermieden werden.

2. Vorrichtung (1) nach Anspruch 1, **dadurch gekennzeichnet, dass** sie ein Laserdatenmodul (112) zum Speichern von Laserparametern des Lasersystems (2) umfasst, und dass das Verarbeitungsmodul (14) eingerichtet ist, die Steuerdaten basierend auf den Eingangsdaten zu erzeugen, wobei die Eingangsdaten zudem die Laserparameter umfassen.

3. Vorrichtung (1) nach einem der Ansprüche 1 oder 2, **dadurch gekennzeichnet, dass** sie ein Augendatenmodul (113) zum Speichern von Augenabmessungen umfasst, und dass das Verarbeitungsmodul (14) eingerichtet ist, die Steuerdaten basierend auf den Eingangsdaten zu erzeugen, wobei die Eingangsdaten zudem die Augenabmessungen umfassen.

4. Vorrichtung (1) nach einem der Ansprüche 1 bis 3, **dadurch gekennzeichnet, dass** sie ein Referenzdatenmodul (114) umfasst zum Speichern von empirischen Daten über Belastungsgrenzen von Augenstrukturen, und dass das Verarbeitungsmodul (14) eingerichtet ist, die Steuerdaten basierend auf den Eingangsdaten zu erzeugen, wobei die Eingangsdaten zudem die Belastungsgrenzen umfassen.

5. Vorrichtung (1) nach Anspruch 4, **dadurch gekennzeichnet, dass** die empirischen Daten Belastungsgrenzen von Augenstrukturen für verschiedene Laserparameter umfassen.

6. Vorrichtung (1) nach einem der Ansprüche 1 bis 5, **dadurch gekennzeichnet, dass** sie ein Modellierungsmodul (141) umfasst, welches eingerichtet ist, basierend auf den Eingangsdaten, sich aus der Behandlung durch den Laserstrahl (21) ergebende Belastungen der Augenstrukturen zu bestimmen, und dass das Verarbeitungsmodul (14) eingerichtet ist, die Steuerdaten basierend auf diesen Belastungen und gespeicherten Belastungsgrenzen zu bestimmen.

7. Vorrichtung (1) nach einem der Ansprüche 1 bis 6, **dadurch gekennzeichnet, dass** das Verarbeitungsmodul (14) eingerichtet ist, die Steuerdaten so zu bestimmen, dass im Gewebe eine mittlere Bestrahlungsstärke reduziert wird und maximale Temperaturen im Gewebe von ausserhalb des Bearbeitungsgebiets liegenden Augenstrukturen, insbesondere in der Iris und der Retina, reduziert werden.

8. Vorrichtung (1) nach Anspruch 7, **dadurch gekennzeichnet, dass** die Steuerdaten Bewegungen des Fokus (F) des Laserstrahls (21) innerhalb des Bearbeitungsgebiets so bestimmen, dass die mittlere Bestrahlungsstärke reduziert wird und die maximalen Temperaturen im Gewebe von ausserhalb des Bearbeitungsgebiets liegenden Augenstrukturen, insbesondere in der Iris und der Retina, reduziert werden.

9. Vorrichtung (1) nach einem der Ansprüche 7 oder 8, **dadurch gekennzeichnet, dass** die Steuerdaten den Laserstrahl (21) zeitweise so aktivieren und deaktivieren, dass die mittlere Bestrahlungsstärke reduziert wird und die maximalen Temperaturen im Gewebe von ausserhalb des Bearbeitungsgebiets liegenden Augenstrukturen, insbesondere in der Iris und der Retina, reduziert werden.

10. Vorrichtung (1) nach einem der Ansprüche 1 bis 9, **dadurch gekennzeichnet, dass** das Verarbeitungsmodul (14) eingerichtet ist, beim Erzeugen der Steuerdaten potentielle Gewebeschäden durch Überhitzung, Phototoxizität und/oder Photokoagulation zu berücksichtigen.

11. Vorrichtung (1) nach einem der Ansprüche 1 bis 10, **gekennzeichnet durch** ein mit dem Lasersystem (2) verbindbares Signalisierungsmodul (142), welches eingerichtet ist, auf den Steuerdaten basierende Steuersignale an das Lasersystem (2) zu übertragen.

12. Vorrichtung (1) nach einem der Ansprüche 1 bis 11, **dadurch gekennzeichnet, dass** das Bearbeitungsdatenmodul (111) mit dem Lasersystem (2) verbindbar ist und eingerichtet ist, die Bearbeitungsdaten direkt vom Lasersystem (2) entgegenzunehmen.

13. Vorrichtung (1) nach einem der Ansprüche 1 bis 12, **gekennzeichnet durch** ein Planungsmodul (143), welches eingerichtet ist, auf den Steuerdaten basierende Bearbeitungsmuster und/oder Bearbeitungsinstruktionen auf einer Anzeige (16) anzuzeigen, welche Bearbeitungsmuster und/oder Bearbeitungsinstruktionen einen Bewegungsablauf des Fokus des Laserstrahls (21) bei der Behandlung des Bearbeitungsgebiets definieren.

14. Vorrichtung (1) nach Anspruch 13, **dadurch gekennzeichnet, dass** das Planungsmodul (143) mit dem Lasersystem (2) verbindbar ist und eingerichtet ist, die Steuerdaten zur Speicherung an das Lasersystem (2) zu übertragen.

15. Vorrichtung (1) nach einem der Ansprüche 2 bis 14, **dadurch gekennzeichnet, dass** die Laserparameter mindestens einen Wert umfassen aus Pulsenergie, Pulsbreite, Pulsrate, Wellenlänge, Fokusgrösse, mittlere Laserleistung, numerische Apertur und Scanmuster.

16. Vorrichtung (1) nach einem der Ansprüche 3 bis 15, **dadurch gekennzeichnet, dass** die Augenabmessungen Angaben über Grösse und relative Position von Augenstrukturen umfassen, wobei die Augenstrukturen mindestens eine aus Cornea, Linse, Retina, Pupille, Bulbus oculi und Iris umfassen.

17. Vorrichtung (1) nach einem der Ansprüche 1 bis 16, **dadurch gekennzeichnet, dass** der Laserstrahl (21) ein von einem Femtolasersystem erzeugter fokussierter gepulster Laserstrahl (21) ist.

18. Computerprogrammprodukt, umfassend Computerprogrammcodemittel zur Steuerung eines oder mehrerer Prozessoren einer Vorrichtung (1) derart, dass die Prozessoren
Bearbeitungsdaten erfassen, die ein bei der Behandlung eines Auges (3) durch den Fokus (F) eines von einem Lasersystem (2) erzeugten gepulsten Laserstrahls (21) zu bearbeitendes Bearbeitungsgebiet definieren, und
Steuerdaten für das Lasersystem (2) bereitstellen, basierend auf Eingangsdaten, wobei die Eingangsdaten die Bearbeitungsdaten umfassen, **dadurch gekennzeichnet, dass** die Prozessoren für die Projektion eines Laserpulses aus den Fokus (F) innerhalb des Bearbeitungsgebiets die Lichtausbreitung im Auge (3) und das in ausserhalb des Bearbeitungsgebiets liegenden Augenstrukturen eingestrahlte Licht ermitteln, und wobei die Prozessoren die Steuerdaten unter Berücksichtigung der ermittelten Lichtausbreitung so bestimmen, dass die Steuerdaten geeignet sind, das Lasersystem (2) so zu steuern, dass eine Behandlung durch den Fokus (F) des Laserstrahls (21) an Positionen innerhalb des Bearbeitungsgebiets zeitlich jeweils so begrenzt ist, dass sich aus der Behandlung durch den Laserstrahl (21) ergebende Gewebeschäden, in ausserhalb des Bearbeitungsgebiets liegenden Augenstrukturen, im wesentlichen vermieden werden.

19. Computerprogrammprodukt nach Anspruch 18, umfassend ein computerlesbares Medium, auf welchem die Computerprogrammcodemittel gespeichert sind.

## Claims

1. Device (1) for protecting tissue in the treatment of an eye (3) with a focused pulsed laser beam (21) generated by a laser system (2), comprising an operating data module (111) for acquiring operating data which define an operating area operated on by the focus (F) of the laser beam (21) during the treatment, wherein the device comprises a processing module (14) for providing control data for the laser system (2) on the basis of input data, the input data comprising the operating data, **characterized in that** the processing module (14) is adapted for determining the light propagation in the eye (3), for the projection of a laser pulse onto the focus (F) within the operating area, and determining the light irradiated into eye structures located outside the operating area, and for determining the control data by taking into consideration the light propagation in the eye in such a manner that the control data limit the time of the treatment by the focus (F) of the laser beam (21) at positions within the operating area in each case in such a manner that tissue damage resulting from the treatment by the laser beam (21), is essentially prevented in eye structures located outside the operating area.

2. Device (1) according to Claim 1, **characterized in that** it comprises a laser data module (112) for storing laser parameters of the laser system (2) and **in that** the processing module (14) is adapted for generating the control data on the basis of the input data, the input data also comprising the laser parameters.

3. Device (1) according to either of Claims 1 and 2, **characterized in that** it comprises an eye data module (113) for storing eye dimensions and **in that** the processing module (14) is adapted for generating the control data on the basis of the input data, the input data also comprising the eye dimensions.

4. Device (1) according to one of Claims 1 to 3, **characterized in that** it comprises a reference data module (14) for storing empirical data about load limits of eye structures and **in that** the processing module (14) is adapted for generating the control data on the basis of the input data, the input data also comprising the load limits.

5. Device (1) according to Claim 4, **characterized in that** the empirical data comprise load limits of eye structures for various laser parameters.

6. Device (1) according to one of Claims 1 to 5, **characterized in that** it comprises a modeling module (141) which is adapted for determining loads on the eye structures resulting from the treatment by the laser beam (21) on the basis of the input data and **in that** the processing module (14) is adapted for determining the control data on the basis of these loads and stored load limits.

7. Device (1) according to one of Claims 1 to 6, **characterized in that** the processing module (14) is adapted for determining the control data in such a manner that a mean irradiation intensity is reduced in the tissue and maximum temperatures in the tissue of eye structures located outside the operating area, particularly in the iris and the retina, are reduced.

8. Device (1) according to Claim 7, **characterized in that** the control data determine movements of the focus (F) of the laser beam (21) within the operating area in such a manner that the mean irradiation intensity is reduced and the maximum temperatures in the tissue of eye structures located outside the operating area, particularly in the iris and retina, are reduced.

9. Device (1) according to either of Claims 7 and 8, **characterized in that** the control data activate and deactivate the laser beam (21) from time to time in such a manner that the mean irradiation intensity is reduced and the maximum temperatures in the tissue of eye structures located outside the operating area, particularly in the iris and the retina, are reduced.

10. Device (1) according to one of Claims 1 to 9, **characterized in that** the processing module (14) is adapted for taking into consideration potential tissue damage due to overheating, phototoxicity and/or photocoagulation when generating the control data.

11. Device (1) according to one of Claims 1 to 10, **characterized by** a signaling module (142) which can be connected to the laser system (2) and which is adapted to transmit control signals based on the control data to the laser system (2).

12. Device (1) according to one of Claims 1 to 11, **characterized in that** the operating data module (111) can be connected to the laser system (2) and is adapted for receiving the operating data directly from the laser system (2).

13. Device (1) according to one of Claims 1 to 12, **characterized by** a planning module (143) which is adapted for displaying operating patterns and/or operating instructions based on the control data on a display (16), which operating patterns and/or operating instructions define a movement sequence of the focus of the laser beam (21) during the treatment of the operating area.

14. Device (1) according to Claim 13, **characterized in that** the planning module (143) can be connected to the laser system (2) and is adapted for transmitting the control data to the laser system (2) for storage.

15. Device (1) according to one of Claims 2 to 14, **characterized in that** the laser parameters comprise at least one value from pulse energy, pulse width, pulse rate, wavelength, focus size, mean laser power, numeric aperture and scanning pattern.

16. Device (1) according to one of Claims 3 to 15, **characterized in that** the eye dimensions comprise information about size and relative position of eye structures, the eye structures comprising at least one of cornea, lens, retina, pupil, eyeball and iris.

17. Device (1) according to one of Claims 1 to 16, **characterized in that** the laser beam (21) is a focused pulsed laser beam (21) generated by a femtolaser system.

18. Computer program product comprising computer program code means for controlling one or more processors of a device (1), in such a manner that the processors acquire operating data which define a processing area to be operated on by the focus (F) of a pulsed laser beam (21) generated by a laser system (2) during the treatment of an eye (3), and
provide control data for the laser system (2) on the basis of input data, the input data comprising the operating data, **characterized in that** the processors determine the light propagation in the eye (3), for the projection of a laser pulse onto the focus (F) within the operating area, and determine the light irradiated into eye structures located outside the operating area, and wherein the processors determine the control data by taking into consideration the light propagation determined, in such a manner that the control data are suitable for controlling the laser system (2) in such a manner that the time of a treatment by the focus (F) of the laser beam (21) at positions within the operating area is limited in each case in such a manner that tissue damage resulting from the treatment by the laser beam (21), is essentially prevented in eye structures located outside the operating area.

19. Computer program product according to Claim 18, comprising a computer-readable medium on which the computer program code means are stored.

## Revendications

1. Dispositif (1) pour protéger des tissus lors du traitement d'un oeil (3) avec un rayon laser (21) pulsé concentré généré par un système à laser (2), comprenant un module de données de traitement (111) destiné à acquérir des données de traitement qui définissent une zone de traitement traitée lors du traitement à travers le foyer (F) du rayon laser (21),
le dispositif comprenant un module de conditionnement (14) destiné à fournir des données de commande pour le système à laser (2) en se basant sur des données d'entrée, les données d'entrée comprenant les données de traitement, **caractérisé en ce que** le module de conditionnement (14) est conçu pour déterminer, pour la projection d'une impulsion laser sur le foyer (F), la propagation de la lumière dans l'oeil (3) à l'intérieur de la zone de traitement ainsi que la lumière injectée dans les structures de l'oeil qui se trouvent à l'extérieur de la zone de traitement, et pour déterminer les données de commande en tenant compte de la propagation de la lumière dans l'oeil de telle sorte que les données de commande limitent à chaque fois dans le temps le traitement à travers le foyer (F) du rayon laser (21) aux positions à l'intérieur de la zone de traitement de telle sorte que les dommages aux tissus qui résultent du traitement par le rayon laser (21) sont pour l'essentiel évités dans les structures de l'oeil qui se trouvent à l'extérieur de la zone de traitement.

2. Dispositif (1) selon la revendication 1, **caractérisé en ce qu'**il comprend un module de données de laser (112) destiné à enregistrer des paramètres de laser du système à laser (2), et que le module de conditionnement (14) est conçu pour générer les données de commande en se basant sur les données d'entrée, les données d'entrée comprenant en plus les paramètres de laser.

3. Dispositif (1) selon l'une des revendications 1 ou 2, **caractérisé en ce qu'**il comprend un module de données d'oeil (112) destiné à enregistrer des dimensions d'oeil, et que le module de conditionnement (14) est conçu pour générer les données de commande en se basant sur les données d'entrée, les données d'entrée comprenant en plus les dimensions d'oeil.

4. Dispositif (1) selon l'une des revendications 1 à 3, **caractérisé en ce qu'**il comprend un module de données de référence (114) destiné à enregistrer des données empiriques sur les limites de contrainte des structures de l'oeil, et que le module de conditionnement (14) est conçu pour générer les données de commande en se basant sur les données d'entrée, les données d'entrée comprenant en plus les limites de contrainte.

5. Dispositif (1) selon la revendication 4, **caractérisé en ce que** les données empiriques comprennent les limites de contrainte des structures de l'oeil pour différents paramètres du laser.

6. Dispositif (1) selon l'une des revendications 1 à 5, **caractérisé en ce qu'**il comprend un module de modélisation (141) qui est conçu, en se basant sur les données d'entrée, pour déterminer les contraintes des structures de l'oeil résultant du traitement par le rayon laser (21), et que le module de conditionnement (14) est conçu pour déterminer les données de commande en se basant sur ces contraintes et sur les limites de contrainte enregistrées.

7. Dispositif (1) selon l'une des revendications 1 à 6, **caractérisé en ce que** le module de conditionnement (14) est conçu pour déterminer les données de commande de telle sorte qu'une intensité d'irradiation moyenne est réduite dans les tissus et que les températures maximales dans les tissus des structures de l'oeil qui se trouvent à l'extérieur de la zone de traitement, notamment dans l'iris et dans la rétine, sont réduites.

8. Dispositif (1) selon la revendication 7, **caractérisé en ce que** les données de commande déterminent les mouvements du foyer (F) du rayon laser (21) à l'intérieur de la zone de traitement de telle sorte que l'intensité d'irradiation moyenne est réduite et les températures maximales dans les tissus des structures de l'oeil qui se trouvent à l'extérieur de la zone de traitement, notamment dans l'iris et dans la rétine, sont réduites.

9. Dispositif (1) selon l'une des revendications 7 ou 8, **caractérisé en ce que** les données de commande activent et désactivent temporairement le rayon laser (21) de telle sorte que l'intensité d'irradiation moyenne est réduite et les températures maximales dans les tissus des structures de l'oeil qui se trouvent à l'extérieur de la zone de traitement, notamment dans l'iris et dans la rétine, sont réduites.

10. Dispositif (1) selon l'une des revendications 1 à 9, **caractérisé en ce que** le module de conditionnement (14) est conçu, lors de la génération des données de commande, pour tenir compte des dommages potentiels des tissus par surchauffe, phototoxicité et/ou photocoagulation.

11. Dispositif (1) selon l'une des revendications 1 à 10, **caractérisé par** un module de signalisation (142) qui peut être relié avec le système à laser (2), lequel est conçu pour transmettre au système à laser (2) des signaux de commande en se basant sur les données de commande.

12. Dispositif (1) selon l'une des revendications 1 à 11, **caractérisé en ce que** le module de données de traitement (111) peut être relié avec le système à laser (2) et est conçu pour accepter les données de traitement directement de la part du système à laser (2).

13. Dispositif (1) selon l'une des revendications 1 à 12, **caractérisé par** un module de planification (143) qui est conçu pour indiquer sur un indicateur (16) un modèle de traitement et/ou des instructions de traitement qui se basent sur les données de commande, ledit modèle de traitement et/ou lesdites instructions de traitement définissant un déroulement du mouvement du foyer du rayon laser (21) lors du traitement de la zone de traitement.

14. Dispositif (1) selon la revendication 13, **caractérisé en ce que** le module de planification (143) peut être relié avec le système à laser (2) et est conçu pour transmettre les données de commande au système à laser (2) en vue de leur enregistrement.

15. Dispositif (1) selon l'une des revendications 2 à 14, **caractérisé en ce que** les paramètres du laser comprennent au moins une valeur parmi : énergie d'impulsion, largeur d'impulsion, fréquence d'impulsion, longueur d'onde, taille du foyer, puissance moyenne du laser, ouverture numérique et modèle de balayage.

16. Dispositif (1) selon l'une des revendications 3 à 15, **caractérisé en ce que** les dimensions de l'oeil contiennent des indications sur la taille et la position relative des structures de l'oeil, les structures de l'oeil comprenant au moins une structure parmi : cornée, lentille, rétine, pupille, globe oculaire et iris.

17. Dispositif (1) selon l'une des revendications 1 à 16, **caractérisé en ce que** le rayon laser (21) est un rayon laser (21) pulsé concentré généré par un système à femtolaser.

18. Programme informatique, comprenant des moyens de code de programme informatique pour commander un ou plusieurs processeurs d'un dispositif (1) de telle sorte que les processeurs
acquièrent des données de traitement qui, lors du traitement d'un oeil (3), définissent une zone de traitement à traiter à travers le foyer (F) d'un rayon laser (21) pulsé généré par un système à laser (2),
fournissent des données de commande pour le système à laser (2) en se basant sur des données d'entrée, les données d'entrée comprenant les données de traitement, **caractérisé en ce que** les processeurs déterminent, pour la projection d'une impulsion laser sur le foyer (F), la propagation de la lumière dans l'oeil (3) à l'intérieur de la zone de traitement ainsi que la lumière injectée dans les structures de l'oeil qui se trouvent à l'extérieur de la zone de traitement, et les processeurs déterminant les données de commande en tenant compte de la propagation déterminée de la lumière de telle sorte que les données de commande conviennent pour commander le système à laser (2) de telle sorte qu'un traitement à travers le foyer (F) du rayon laser (21) aux positions à l'intérieur de la zone de traitement est à chaque fois limité dans le temps de telle sorte que les dommages aux tissus qui résultent du traitement par le rayon laser (21) sont pour l'essentiel évités dans les structures de l'oeil qui se trouvent à l'extérieur de la zone de traitement.

19. Programme informatique selon la revendication 18, comprenant un support lisible par ordinateur sur lequel sont enregistrés les moyens de code de programme informatique.
